# EUROPEAN PATENT SPECIFICATION

(11) **EP 4 188 402 B1**
(45) Date of publication and mention of the grant of the patent: **04.12.2024**
(21) Application number: 21752596.3
(22) Date of filing: 29.07.2021
(51) Int. Cl.: A61K 35/745, A61K 35/747, A61P 17/00, A61P 17/10

(54) **PROBIOTIC COMPOSITIONS AND THEIR USE IN THE PREVENTION AND/OR TREATMENT OF SKIN DISEASES AND/OR DISORDERS**
PROBIOTISCHE ZUSAMMENSETZUNGEN UND DEREN VERWENDUNG BEI DER VORBEUGUNG UND/ODER BEHANDLUNG VON HAUTERKRANKUNGEN UND/ODER -ERKRANKUNGEN
COMPOSITIONS PROBIOTIQUES ET LEUR UTILISATION DANS LA PRÉVENTION ET/OU LE TRAITEMENT DE MALADIES ET/OU DE TROUBLES CUTANÉS

(30) Priority: 31.07.2020 IT 202000018901
(43) Date of publication of application: 07.06.2023
(73) Proprietor: SYNBALANCE SRL, 21040 Origgio (VA) (IT)
(72) Inventor: MALANCHIN, Rosella, 21040 Origgio (VA) (IT); PIANGIOLINO, Cristiana, 21040 Origgio (VA) (IT); CASTEGNARO, Silvia, 21040 Origgio (VA) (IT); CARLOMAGNO, Federica, 21040 Origgio (VA) (IT); PESCIAROLI, Chiara, 21040 Origgio (VA) (IT)
(74) Representative: Bianchetti & Minoja with Trevisan & Cuonzo IPS SRL
(86) International application number: PCT/IB2021/056913
(87) International publication number: WO 2022/024027

(56) References cited:
- WO-A1-2011/110918
- WO-A2-2017/025936
- US-A1- 2009 169 531
- FENG JUE-RONG ET AL: "Efficacy and safety of probiotic-supplemented triple therapy for eradication ofHelicobacter pyloriin children: a systematic review and network meta-analysis", EUROPEAN JOURNAL OF CLINICAL PHARMACOLOGY, SPRINGER VERLAG, DE, vol. 73, no. 10, 5 July 2017 (2017-07-05), pages 1199 - 1208, XP036319418, ISSN: 0031-6970, [retrieved on 20170705], DOI: 10.1007/S00228-017-2291-6
- I. PRESTI ET AL: "Evaluation of the probiotic properties of new Lactobacillus and Bifidobacterium strains and their in vitro effect", APPLIED MICROBIOLOGY AND BIOTECHNOLOGY, vol. 99, no. 13, 7 March 2015 (2015-03-07), pages 5613 - 5626, XP055200124, ISSN: 0175-7598, DOI: 10.1007/s00253-015-6482-8

## Description

### Field of the invention

It is disclosed a probiotic composition comprising *Lactobacillus plantarum*, *Lactobacillus rhamnosus* and *Lactobacillus reuteri*, useful in the prevention and/or treatment of skin diseases and/or disorders.

### Background of the invention

Skin is one of the largest organs in the human body. Skin is prone to diseases and disorders of various etiologies, such as, for example, atopic dermatitis and acne.

Atopic dermatitis (AD) is a multifactorial allergic skin disease with a high worldwide incidence in both adults and children. Atopic dermatitis nature is inflammatory and chronic, and induce itching, with a wide scale of severity, a high rate of relapses and infections due to rubbing, with a serious impact on the quality of life of the subject. Symptoms often associated with atopic dermatitis are: itching, redness, recurrent eczematous lesions, dry and squamous skin. In recent decades, the incidence of atopic dermatitis at worldwide level has increased: about 60% of the population is affected, especially children (60% in the first year of life and 85% before the age of 5). Generally, it is a typical disorder of early childhood, which tends to disappear during adolescence, but in some cases it persists even in adulthood. There are several treatments for atopic dermatitis, some of them are palliatives such as the daily use of emollients and ceramides, to reduce trans-epidermal water loss, while others are prescribed to treat and prevent relapses, such as non-specific corticosteroids.

Recently, has been approved a therapy based on monoclonal antibodies for the most acute cases of atopic dermatitis: Dupilumab, which is an injectable drug based on human IgG4 monoclonal antibodies able to inhibit the response of the cytokines IL-4 and IL-13 with promising results. However, its use remains under debate due to its scarce applicability as a routine treatment.

Although the use of anti-inflammatory drugs is the most prescribed treatment, a large concern is recently arising due to the high rate of side effects associated with them. In addition, corticosteroids are not suitable for treating delicate parts of the body, such as the eyelids, and are highly discouraged in early childhood. Some studies have highlighted an improvement with reduction in disease severity after a minimum of eight weeks intake of a probiotic complex, but the scientific evidence demonstrating their effectiveness is not solid enough.

Acne is a multifactorial disorder with inflammatory prevalence, which affects parts of the skin with a high concentration of pilosebaceous units, structures composed of hair bulbs associated with sebum-producing sebaceous glands. Acne can manifest itself through different types of lesions divided into open (blackheads) or closed (whiteheads) comedones, cysts, papules, pustules or nodules, following obstruction and inflammation of the pilosebaceous units; in addition, excess of sebum makes the affected area sensitive to *P. acnes* infections. The areas where acne occurs most frequently are the face, back and chest, with negative consequences on the subject's quality of life.

The incidence of acne is high: it affects mostly in adolescence - puberty, about 83% of adolescents, but also in adulthood, especially in the female gender (about 20%). To treat acne, many therapies are available, divided in topical or systemic treatments aiming to reduce the production of sebum, the formation of new comedones, the inflammation, the bacterial load and to normalize the keratinization process.

In milder cases, retinoid or topical antibiotics are preferred, while in more severe cases the oral intake of antibiotics or isotretinoin is recommended. However, several evidence show that antibiotics are not effective against a severe form of acne: in these cases, isotretinoin, a retinoid drug derived from vitamin A, is preferred, since it is the treatment that gives a significant improvement of the condition. Despite the important action of the drug on the disease, several side effects are known at mucosal level (dry mouth, nasal, and ocular) and at liver level with bioaccumulations of retinoid that can cause hypervitaminosis of vitamin A with permanent liver and spleen deficiency. The presence of side effects is not exclusively associated with isotretinoin, but also to most existing pharmacological treatments.

The use of topical retinoids can cause skin irritation, dryness and burning, as well as the application of benzoyl peroxide; antibiotic therapy, although recommended, is not considered the best strategy against acne since this pathology is 78% inflammatory and the use of antibiotics for a long period could have a more negative than positive impact for acne and for the alteration of the intestinal and skin microbiota.

In light of the above, there is still a need to find effective and safe alternative solutions in the prevention and/or treatment of chronic skin diseases and conditions.

### Summary of the invention

The present invention concerns probiotic compositions including *Lactobacillus plantarum, Lactobacillus rhamnosus* and *Lactobacillus reuteri.* Moreover, the invention relates to the use of said composition in the prevention and/or treatment of skin diseases and/or disorders.

### Detailed description of the invention

Surprisingly, it has been found that compositions comprising a probiotic combination with at least one (one or more) strain belonging to each of the three species: *Lactobacillus plantarum, Lactobacillus rhamnosus* and *Lactobacillus reuteri,* are effective in the prevention and/or treatment of skin diseases, in particular inflammatory, autoimmune or allergic skin diseases.

It is disclosed a composition comprising three different probiotic species: *Lactobacillus plantarum* (also known as: *Lactiplantibacillus plantarum*), *Lactobacillus rhamnosus* (also known as: *Lacticaseibacillus rhamnosus*) and *Lactobacillus reuteri* (also known as: *Limosilactobacillus reuteri*), according to the present invention the compositions comprise a probiotic combination comprising *Lactobacillus plantarum* PBS067, *Lactobacillus rhamnosus* LRH020 and *Lactobacillus reuteri* PBS072.

According to a preferred aspect of the invention, the compositions comprise a probiotic combination of a preferred strain for each of the three different probiotic species, in particular the compositions may comprise *Lactobacillus plantarum* PBS067, *Lactobacillus rhamnosus* LRH020 and *Lactobacillus reuteri* PBS072.

According to a further aspect of the invention, in the compositions one of the three strains belonging to one of the three species: *Lactobacillus plantarum* PBS067, *Lactobacillus rhamnosus* LRH020 or *Lactobacillus reuteri* PBS072 may be replaced with a strain belonging to the *Lactobacillus acidophilus* species, preferably with the *Lactobacillus acidophilus* strain PBS066.

*Lactobacillus plantarum* strain (*L. plantarum* or *LP*) called "PBS067" was deposited at the Leibniz-Institut DSMZ-Deutsche Sammlung von Mikroorganismen und Zellkulturen GmbH, under the Budapest Treaty, on 17/06/2011, under Accession Number "DSM 24937".

*Lactobacillus rhamnosus* strain (*L. rhamnosus* or *LRH*) called "LRH020" was deposited at the Leibniz-Institut DSMZ-Deutsche Sammlung von Mikroorganismen und Zellkulturen GmbH, under the Budapest Treaty, on 17/01/2012, under Accession Number "DSM 25568".

*Lactobacillus reuteri* strain (*L. reuteri or LR*) called "PBS072" was deposited at the DSMZ - Deutsche Sammlung von Mikroorganismen und Zellkulturen GmbH under the Budapest Treaty, on 14/092011, under Accession Number "DSM 25175".

*Lactobacillus acidophilus* strain (*L. acidophilus* or *LA*) called "PBS066" was deposited at the DSMZ - Deutsche Sammlung von Mikroorganismen und Zellkulturen GmbH under the Budapest Treaty, on 17/06/2011, under Accession Number "DSM 24936".

The present invention relates to compositions comprising, as active ingredients, a probiotic combination comprising, preferably consisting of, *Lactobacillus plantarum* PBS067, *Lactobacillus rhamnosus* LRH020 and *Lactobacillus reuteri* PBS072, in admixture with at least one physiologically acceptable excipient and/or vehicle.

Compositions may be formulated by conventional methods. Preferred forms of administration are solid formulations, such as hard capsules, sachets, sticks, oro-soluble sticks, tablets, granules, or liquid formulations, such as vials with dosing cap mono- or multi-dose, multi-dose dispersions in the oily phase, drops, syrups, multi-phase emulsions, etc.

Further object of the present invention is the use of probiotic compositions comprising *Lactobacillus plantarum* PBS067, *Lactobacillus rhamnosus* LRH020 and *Lactobacillus reuteri* PBS072, in the prevention and/or treatment of skin diseases and/or disorders, in particular of the inflammatory, allergic or autoimmune type.

The strain of *Lactobacillus plantarum* (LP) PBS067, may be present in the composition in a percentage by weight on the total weight of the probiotic combination from 10% to 30%, preferably from 15% to 25%, even more preferably it is equal to 20%.

The strain of *Lactobacillus plantarum* (LP) PBS067, may be present in each individual unit dose in amounts ranging from 0,5 to 2,5 billion CFU, preferably in amounts ranging from 1 to 2 billion CFU, more preferably it is present in amount of 1 billion CFU.

The strain of *Lactobacillus rhamnosus* (LRH) LRH020, may be present in the composition in a percentage by weight on the total weight of the probiotic combination from 25% to 45%, preferably from 30% to 40%, even more preferably it is equal to 35%.

The strain of *Lactobacillus rhamnosus* (LRH) LRH020 may be present in each individual unit dose in amounts ranging from 0,5 a 2,5 billion CFU, preferably in amounts ranging from 1 to 2 billion CFU, more preferably it is present in amount of 1 billion CFU.

The strain of *Lactobacillus reuteri* (LR) PB S072, may be present in the composition in a percentage by weight on the total weight of the probiotic combination from 35% to 55%, preferably from 40% to 50%, even more preferably it is equal to 45%.

The strain of *Lactobacillus reuteri* (LR) PBS072 may be present in each individual unit dose in amounts ranging from 0,5 a 2,5 billion CFU, preferably in amounts ranging from 1 to 2 billion CFU, more preferably it is present in amount of 1 billion CFU.

The three species of *Lactobacillus plantarum* PBS067, *Lactobacillus rhamnosus* LRH020 and *L. reuteri* PBS072 may be present in a weight ratio of 1:2,5: 1,75 or 1: 1: 1 ratio if expressed as CFU.

Probiotic compositions of the invention can be administered orally or topically.

According to a preferred aspect, inflammatory skin diseases and/or disorders are selected from the group comprising: dermatitis, such as seborrheic dermatitis; rosacea; acne; alopecia; cutaneous vasculitis; eczema; urticaria; angioedema; erythema; dandruff; vitiligo; preferably from the group comprising: rosacea; acne; cutaneous vasculitis; eczema; hives; angioedema; erythema; dandruff; more preferably the inflammatory skin disease and/or disorder is acne.

According to another preferred aspect, allergic skin diseases and/or disorders are selected from the group comprising: dermatitis, such as atopic dermatitis, contact dermatitis or photoallergies from contact; eczema; allergic hives; angioedema; erythema; preferably the allergic skin disease and/or disorder is atopic dermatitis.

Autoimmune skin diseases and/or disorders may be selected preferably from the group comprising: psoriasis; systemic, subacute and chronic lupus erythematosus; scleroderma; alopecia; dermatomyositis; vulgar or bullous pemphigus; herpetiform dermatitis; linear IgA dermatitis; autoimmune urticaria; vitiligo; more preferably from the group comprising: psoriasis; herpetiform dermatitis; autoimmune urticaria.

According to a further preferred aspect, skin diseases and/or disorders are selected from acne, atopic dermatitis, psoriasis, rosacea, scleroderma, pemphigus vulgaris or bullous, vitiligo, alopecia, urticaria.

The following examples further illustrate the invention.

### Examples

### Formulative examples

### Example 1

A composition in powder form in capsules has been prepared, containing:

| **INGREDIENTS** | **% p/p** | **BILLIONS CFU/DOSE** |
|---|---|---|
| *Lactobacillus plantarum* PBS067 | 14 | 1 |
| *Lactobacillus rhamnosus* LRH020 | 24 | 1 |
| *Lactobacillus reuteri* PBS072 | 35 | 1 |
| Corn starch | 26 | - |
| Magnesium stearate vegetable | 1 | - |

### Example 2

A composition in powder form in capsules has been prepared, containing:

| **INGREDIENTS** | **% p/p** | **BILLIONS CFU/DOSE** |
|---|---|---|
| *Lactobacillus plantarum* PBS067 | 10 | 1 |
| *Lactobacillus rhamnosus* LRH020 | 18 | 1 |
| *Lactobacillus reuteri* PBS072 | 26 | 1 |
| Maltodextrin | 43.96 | - |
| Magnesium stearate vegetable | 1 | - |
| Silicon dioxide | 1 | - |
| D-Biotin | 0.04 | - |

### Example 3

A composition in powder form in water-soluble stick has been prepared, containing:

| **INGREDIENTS** | **% p/p** | **BILLIONS CFU/DOSE** |
|---|---|---|
| *Lactobacillus plantarum* PBS067 | 0.9 | 1 |
| *Lactobacillus rhamnosus* LRH020 | 1.6 | 1 |
| *Lactobacillus reuteri* PBS072 | 2.3 | 1 |
| Maltodextrin | 86.2 | - |
| FOS | 6.7 | - |
| Flavour | 1.3 | - |
| Silicon dioxide | 1 | - |

### Example 4

A composition in form of monodose vial with a dosing cap has been prepared, containing:

| **INGREDIENTS** | **% p/p** | **BILLIONS CFU/DOSE** |
|---|---|---|
| **Solid phase- Cap** | | |
| *Lactobacillus plantarum* PBS067 | 4 | 1 |
| *Lactobacillus rhamnosus* LRH020 | 7 | 1 |
| *Lactobacillus reuteri* PBS072 | 11 | 1 |
| FOS 93% | 39 | - |
| Inulin | 39 | - |

| **Liquid phase (10 mL)** | | |
|---|---|---|
| Purified water | 99.1 | |
| Potassium Sorbate | 0.1 | |
| Sodium Benzoate | 0.1 | |
| Flavour | 0.2 | |
| Acidifier: citric acid | 0.3 | |
| Sweetener | 0.2 | |

### Example 5

A composition in powder form in oro-soluble stick has been prepared, containing:

| **INGREDIENTS** | **% p/p** | **BILLIONS CFU/DOSE** |
|---|---|---|
| *Lactobacillus plantarum* PBS067 | 0.93 | 1 |
| *Lactobacillus rhamnosus* LRH020 | 1.56 | 1 |
| *Lactobacillus reuteri* PBS072 | 2.33 | 1 |
| Maltodextrin | 76.40 | - |
| Sorbitol | 9.00 | - |
| FOS 93% | 3.60 | - |
| Inulin 90% | 3.73 | - |
| Flavour | 1.30 | - |
| Silicon dioxide | 1.00 | - |
| Folic Acid | 0.01 | - |
| Vitamin B12 | 0.04 | - |
| Vitamin B6 | 0.02 | - |
| Sucralose | 0.08 | - |

### Example 6

A composition in form of suspension in oily phase has been prepared, containing:

| **INGREDIENTS** | **% p/p** | **BILLIONS CFU/DOSE** |
|---|---|---|
| **Solid phase** - **cap** | | |
| *Lactobacillus plantarum* PBS067 | 6.0000 | 1 |
| *Lactobacillus rhamnosus* LRH020 | 10.0000 | 1 |
| *Lactobacillus reuteri* PBS072 | 15.0000 | 1 |
| Maltodextrin | 69.0000 | - |

| **Liquid phase (5 mL)** | | |
|---|---|---|
| Sunflower oil | 60.0000 | - |
| Medium-Chain Triglycerides (MCT) | 38.9990 | - |
| Silicon dioxide | 1.0000 | - |
| Vitamin D3 | 0.0010 | - |

### Example 7

A composition in form of body anhydrous stick has been prepared:

| **INGREDIENTS** | **% p/p** | **BILLIONS CFU/DOSE** |
|---|---|---|
| *Lactobacillus plantarum* PBS067 | 0.0010 | 0.5 |
| *Lactobacillus rhamnosus* LRH020 | 0.0017 | 0.5 |
| *Lactobacillus reuteri* PBS072 | 0.0025 | 0.5 |
| *Helianthus Annuus* Seed Wax. Synthetic Wax | 10.0000 | - |
| *Helianthus Annuus* Seed Wax | 3.5000 | - |
| Synthetic Beeswax | 7.0000 | - |
| Triolein. Glyceryl Dioleate | 50.5948 | - |
| Tripelargonin | 10.6000 | - |
| *Butyrospermum Parkii* Butter | 12.0000 | - |
| Olive Glycerides, Ceramide NP | 1.0000 | - |
| Polyglyceryl-2 Isostearate/ Dimer Dilinoleate Copolymer | 5.0000 | |
| *Brassica Campestris* Seed oil, Tocopherol, *Rosmarinus Officinalis* Leaf Extract | 0.1000 | - |
| Flavour | 0.2000 | - |

### Experimental examples

The following experimental examples relates to the evaluation of the effectiveness of the compositions of the invention in the treatment of atopic dermatitis and acne.

### Example 8 - In-vitro assays

In-vitro assays have been carried out to evaluate *L. acidophilus* PBS066 probiotic strain in preventing and/or treating skin disorders.

### Modulation of IL-4 cytokine

IL-4 is an anti-inflammatory cytokine and its concentration tends to increase during an inflammatory response.

### Materials and methods

The viability of cell cultures (BALB/c3T3, clone A31) treated with SDS (sodium dodecyl sulfate, irritant/pro-inflammatory agent - exposure time: 24 hours and 5 days) in the presence and absence of probiotics has been assessed.

The results are compared with those obtained for positive control cells treated exclusively with SDS (CTR+) and negative control cells, without any treatment (CTR-).

### Results

*L. acidophilus* PBS066 strain has been shown to significantly increase the concentration of anti-inflammatory cytokine in both an acute condition: 24 hours, and chronic: 5 days (Table 1).

**TABLE 1**

| ***IL-4*** | ***24h*** | ***5d*** |
|---|---|---|
| *CTR-* | *516.5* | *273.9* |
| *CTR*+ | *584.2* | *335.0* |
| *LA* - *PBS066* | *631.4* | *449.2* |

### Modulation of Tumor Necrosis Factor α (TNF-α)

TNF-α is the first pro-inflammatory cytokine recruited during the initiation of a type 1 immune response.

### Materials and methods

Viability of cell cultures (BALB/c3T3, clone A31) treated with SDS (sodium dodecyl sulphate, irritant/pro-inflammatory agent - exposure time: 24 hours and 5 days) in the presence and absence of *L. acidophilus* PBS066 has been assessed. The results are compared with those obtained for positive control cells treated exclusively with SDS (CTR+) and negative control cells, without any treatment (CTR-).

### Results

*L. acidophilus* PBS066 strain has been shown to significantly reduce the concentration of the pro-inflammatory cytokine in the acute phase. This finding is in line with cell physiology considering that TNF-α is one of the cytokines involved in the acute phase of inflammatory response (Table 2).

**TABLE 2**

| ***TNF-a*** | ***24h*** | ***5d*** |
|---|---|---|
| *CTR-* | *139.7* | *932.3* |
| *CTR*+ | *406.2* | *2706.9* |
| *LA* - *PBS066* | *149.1* | *2513.4* |

### Modulation of cell antioxidant capacity

The antioxidant power is measured to observe the ability of cells to respond to conditions of strong oxidative stress.

### Materials and methods

The antioxidant and reducing capacity of cell cultures treated with the *L. acidophilus* PBS066 strain has been evaluated.

The assessment was carried out using the FRAP test, which is the ability of cells to reduce (oxidized) trivalent iron to (reduced) bivalent iron. Iron is an oxidizing agent in this experimental system because of its transition metal nature and therefore an agent capable of inducing oxidative stress.

### Results

*L. acidophilus* PBS066 strain proved to greatly increase the antioxidant power of the cell both in an acute condition: 24 hours, and chronic: 5 days (Table 3).

**TABLE 3**

| ***FRAP*** | ***24H*** | ***5D*** |
|---|---|---|
| *CTR-* | *160.2* | *346.2* |
| *LA* - *PBS066* | *491.7* | *242.1* |

### Modulation of sensitization markers

Allergens are able to induce phenotypic alterations in dendritic cells, increase both the production of cytokines involved in the allergic response and the number of receptors (CD54 or CD86) on the cell surface responsible for activating T cells towards an allergic response.

### Materials and methods

The in-vitro probiotic ability in modulating the number of CD54 and CD86 receptors present on the surface of THP1 monocytes, was evaluated by treatment of the cell line with a sensitizing agent (2,4-dinitroclorobenze -DNCB). Subsequently, the fluorescence index of the individual strains compared with the negative control (system not disturbed by DNCB) and positive (system disturbed, but in the absence of probiotics) was measured through the flow cytometer.

### Results

*L. acidophilus* PBS066 strain has been shown to significantly inhibit the action of sensitization markers by positive modulation of expression of CD54 and CD86 receptors (Tables 4 and 5):

**TABLE 4**

| ***CD54*** | ***RFI %*** |
|---|---|
| *CTR-* | *100.00* |
| *CTR*+ | *272.33* |
| *LA* - *PBS066* | *174.33* |

**TABLE 5**

| ***CD86*** | **RFI %** |
|---|---|
| *CTR-* | 100.00 |
| *CTR*+ | 197.00 |
| *LA* - *PBS066* | 142.67 |

### Modulation of Beta-Defensin 2 and Cathelicidin peptides

Beta-Defensin and Cathelicidin are peptides involved in the innate immune response to protect the human body from infections. They act locally in the skin district as the first barrier against infection in the area of a skin lesion by pathogens.

### Materials and methods

*L. acidophilus* PBS066 strain has been evaluated for its anti-inflammatory and immunomodulation activity in vitro in Caco2 cells, after induction of inflammatory stress by lipopolysaccharide (LPS). Immunomodulation activity was measured by the expression of Beta Defensin-2 (DEFB2) and Cathelicidin Antimicrobial Peptide (CAMP) after induction of inflammation in the presence of PBS066. The results were compared with positive and negative control (CTR- as cells without any stress condition, CTR+ as cells subject to stress induction LPS).

### Results

Results showed that *L. acidophilus* PBS066 strain is able to positively modulate the selected markers, increasing the production, in the Caco2 cell line, of Beta-Defensin and Cathelicidin as shown in Tables 6 and 7.

**TABLE 6**

| | ***DEFB2*** |
|---|---|
| CTR- | 4.68 |
| CTR+ | 5.18 |
| LA - PBS066 | 5.99 |

**TABLE 7**

| | ***CAMPS*** |
|---|---|
| CTR- | 11.90 |
| CTR+ | 12.63 |
| LA - PBS066 | 14.01 |

### Antimicrobial activity

It has been observed that infections by *S. aureus, S. epidermidis* and *P. acnes* are common in people with skin disorders.

*L. acidophilus* PBS066 strain has been shown in vitro to have a strong antimicrobial activity against these pathogens.

The results showed that the strain of *L. acidophilus* PBS066 has considerable capacities both in a "preventive" model - probiotic plate growth and subsequent overlay of the pathogen strain - and in the "treatment" model - plate growth of the pathogen followed by addition of the culture medium of post-biotic enriched probiotic strains released during fermentation (Tables 8, 9 and 10).

Legend: ** strong inhibition; * moderate inhibition; - no inhibition.

**TABLE 8**

| **Probiotic strain** | **Preventive model** | **Treatment model** |
|---|---|---|
| | ***S. aureus*** | ***S. aureus*** |
| **LA -PBS066** | ****** | ***** |

**TABLE 9**

| **Probiotic strain** | **Preventive model** | **Treatment model** |
|---|---|---|
| | ***S. epidermidis*** | ***S. epidermidis*** |
| **LA -PBS066** | ***** | ***** |

**TABLE 10**

| **Probiotic strain** | **Preventive model** | **Treatment model** |
|---|---|---|
| | ***P. acnes*** | ***P. acnes*** |
| **LA -PBS066** | - | ****** |

### Example 9 - Atopic Dermatitis

A clinical study was carried out to assess the oral efficacy of the probiotic composition of Example 1 compared to the placebo formulation of Table 11 reported below in the treatment of atopic dermatitis in adults.

### Material and methods

A single centre, randomized, double-blind, placebo-controlled, parallel group study was carried out.

Eighty subjects of both sexes, aged between 18 and 50 years and showing a mild-moderate score of atopic dermatitis (AD) diagnosed by SCORAD, were enrolled and subsequently included only if they met the inclusion criteria of the study. Subjects were equally assigned to two groups (40 subjects in the active group and 40 in the placebo group) according to a randomization list, to received one capsule/day of composition of Example 1 or placebo (Table 11) for 56 days.

**TABLE 11**

| **INGREDIENTS** | **% p/p** |
|---|---|
| Corn Starch | 99 |
| Magnesium stearate vegetable | 1 |

Subjects were also supplied with a moisturizing cream, to be used throughout the study period in substitution of their usual body cream.

Clinical visits were scheduled as follow: initial visit (T0d), intermediate (T28d) and final visit (T56d); moreover, a follow-up visit was planned 28 days after the last products intake (T84d).

During the visits, the following parameters were assessed: clinical evaluation of the correct use of the product, clinical, instrumental and biochemical evaluations such as "skin tape stripping" to measure the concentration of inflammatory cytokines. Finally, during the end-of-treatment visit and 28 days after the end of product intake, the subjects were given a questionnaire to assess their personal perception of the treatment.

### Assessment of Clinical Effects

All clinical parameters were assessed by a dermatologist under controlled room conditions (T=22±2°C and RH=40-60%) after 15-20 minutes of acclimatization.

AD severity was evaluated according to SCORAD (SCORing AD) index (as developed by European Task Force on Atopic Dermatitis (ETFAD).

Skin smoothness was clinically evaluated in accordance with the clinical scores reported in Table 12.

**TABLE 12**

| **Skin smoothness level** | **Score** |
|---|---|
| Not smooth | **1** |
| A little smooth | **2** |
| Smooth | **3** |
| Clearly smooth | **4** |

Skin moisturization was measured according to the Corneometer^{®} method, using Corneometer^{®} CM 825 (Courage+Khazaka, electronic GmbH).

Transepidermal water loss (TEWL) was measured indirectly using a Tewameter^{®} TM 300 (Courage+Khazaka, electronic GmbH).

Skin reactions, both physical (erythema, oedema, dryness and desquamation) and functional (tightness, itching and burning), scored according to a points scale (0. None; 1. Very Mild; 2. Mild; 3. Moderate; 4. Severe) were evaluated each time a sign (physical or functional) appeared.

Subjects were asked to express their personal opinion on the treatment by answering to a questionnaire about the product acceptability and efficacy at the end of the treatment period and at the end of the follow-up period.

### Digital macrophotography and skin tape stripping

Pictures of the skin areas affected by atopic dermatitis were acquired from each subjects at each clinical visit (T0d, T28d, T56d, T84d) using a digital professional reflex camera (NIKON 0300 digital camera).

Skin tape stripping were sampled using Corneofix^{®} (Courage+Khazaka) under temperature, relative humidity and pressure controlled conditions: 10 consecutively strips in the area closed to the one selected were collected. Skin stripping were stored at -80°C until extraction with phosphate buffer solution + Triton 1%: cytokines Tumor Necrosis Factor alpha (TNF-alpha), Thymus- and activation-regulated chemokine (TARC) and Thymic stromal lymphopoietin (TSLP) were determined by using commercially available kits ELISA (RayBio^{®} Human TSLP ELISA, RayBio^{®} Human TARC (CCL17) ELISA, BosterBio Human TNF-Aipha ELISA).

### Statistical analysis

The collected data were summarized using frequency distributions (number and percentage) for categorical/ordinal variables. For continuous variables the following values were calculated: mean value, minimum value, maximum value, standard error of the mean (SEM), individual variation/individual percentage variation, mean variation/mean percentage variation. All the calculations were done using a Microsoft^{®} Excel 2013 (vers. 15.0.4885.1001; Microsoft, USA) worksheet running on Microsoft^{®} Windows 8.1 Professional (Microsoft, USA).

The results of self-assessment questionnaire were calculated as percentage (%) of subjects who assigned a particular judgment (among those proposed). For each question, the number of subjects related to each judgment was counted - (number of subjects) and then divided by the total number of subjects.

### Statistical methods

The instrumental data were submitted to ANOVA test followed by Tukey-Kramer post-test (intragroup analysis); the inter-group statistical analysis was performed on the data variations versus T0d by means of Bilateral Student's Test t for unpaired data. The clinical data were analysed using Mann-Whitney U / Wilcoxon Rank-Sum Test (Two Samples). The statistical software used for statistical analyses was: NCSS 10 statistical software (NCSS, LLC. Kaysville, Utah, USA) running on Windows Server 2008 R2 Standard (Microsoft, USA).

A p value <0.05 was considered statistically significant for all analyses.

### Results

Treatments with the probiotic composition of Example 1 and placebo were well tolerated by all the subjects and no adverse events were reported during the study period; furthermore, no drop-out occurred. There were no significant differences between the probiotic composition of Example 1 and placebo group in any of the baseline characteristics including age, gender and initial SCORAD score (Table 13), confirming the unbiased randomization.

**TABLE 13**

| | AC | PL |
|---|---|---|
| Sex | | |
| Male | 7 | 5 |
| Female | 33 | 35 |
| Age (± SEM) | 39 ± 1.8 | 38 ± 1.4 |
| SCORAD (± SEM) | 20.9 ± 0.5 | 19.7 ± 0.4 |

Products treatment resulted in an overall improvement in the SCORAD index in both groups; however, a statistically significant and progressive decrease of the SCORAD index was measured in the group treated with the probiotic composition of Example 1 throughout the administration period (from 20.9±0.5 at T0d to 16.9±0.5 at T28d and to 13.7±0.6 at T56d); moreover, the amelioration of the SCORAD index was measured even after 28 days of probiotic product intake discontinuation (14.8±0.6 at T84d).

The probiotic composition of Example 1 treatment resulted in significantly lower SCORAD index, throughout the study period, compared to those obtained with the placebo (p<0.001 vs baseline and vs placebo) and at the beginning of treatment (p<0.001 vs baseline vs placebo) as shown in Table 14.

**TABLE 14**

| | T0 | T28 | T56 | T84 |
|---|---|---|---|---|
| Active | 20.9 ± 0.5 | 16.9±0.5***^{###} | 13.7±0.56***^{###} | 14.8±0.57***^{###} |
| Placebo | 19.7 ± 0.4 | 18.4 ± 0.5 | 17.3 ± 0.62 | 17.6 ± 0.59 |

| | | | | |
|---|---|---|---|---|
| Legend: *** Tukey-Kramer's p<0.001 vs T0 ^{###} p<0.001 Bilateral Student's Test t for the independent variables (variation of the data compared to T0) Active vs Placebo | | | | |

Clinical evaluation of skin smoothness resulted in a similar profile: the group treated with the probiotic composition of Example 1 showed a progressive increment in the percentage of subjects with amelioration of skin smoothness in respect to the beginning of the study, 57.5% at T28d and 82.5% at T56d, and a sustained percentage (77.5%) at T84d. Such improvement was statistically significant compared to baseline as well as to the placebo group (Table 15).

**TABLE 15**

| | T28 | T56 | T84 |
|---|---|---|---|
| Active | 57.5% ***^{##} | 82.5% ***^{##} | 77.5% ***^{##} |
| Placebo | 15.0% | 37.5% ** | 30.0% ** |

| | | | |
|---|---|---|---|
| Legend: * Wilcoxon test p<0.05 vs T0 ** Wilcoxon test p<0.01 vs T0 *** Wilcoxon test p<0.001 vs T0 ^{##} p<0.01 Mann-Whitney U Test for placebo comparisons | | | |

A global enhancement of skin moisturization was also recorded, as a reduction of Trans Epidermal Water Loss (TEWL) and an increase of the skin moisturization indexes measured by the corneometer: TEWL showed a statistically significant reduction, compared to baseline, at T28d (-9.5%) and at T56d (-19.3%), and it was still reduced 28 days after the last probiotics intake (-15.0%). Moreover, the TEWL reduction was statistically significant at all the study times also in respect to the placebo (Table 16).

**TABLE 16**

| | T0 | T28 | T56 | T84 |
|---|---|---|---|---|
| Active | 14.6 ± 1.3 | 13.2 ± 1.1 (-9.5%) *^{##} | 11.6 ± 1.0 (-19.3%)***^{###} | 12.0 ± 1.0 (-15.0%)***^{###} |
| Placebo | 12.8 ± 1.1 | 12.2 ± 0.9 (-3.1%) | 11.7 ± 0.9 (-6.0%)** | 12.0 ± 0.9 (-2.8%) *** |

In brackets are reported the percentage variation with respect to the T0 (i.e. [Tₓ-T₀]/T₀).

Legend:
* Tukey-Kramer's p<0.05 vs T0 ** Tukey-Kramer's p<0.01 vs T0
*** Tukey-Kramer's p<0.001 vs T0
^{##} p<0.01 - ^{###} p<0.001 Bilateral Student's Test t for the independent variables (carried out on the variation of the data with respect to T0) Active vs Placebo

A similar trend was observed also for skin moisturization: the active group exhibited a progressive and significant increase of such parameter throughout the administration of the probiotic composition of Example 1 (22.9% at T28d, 33.7% at T56d, and 28.3% at T84d); and a statistically significant difference (p<0.001) in respect to the placebo group was appreciated at all the study times (Table 17).

**TABLE 17**

| | T0 | T28 | T56 | T84 |
|---|---|---|---|---|
| Active | 23.1 ± 0.9 | 28.5 ± 1.3 (22.9%) ***^{###} | 30.8 ± 1.3 (33.7%) ***^{###} | 29.4 ± 1.2 (28.3%) ***^{###} |
| Placebo | 24.7 ± 1.1 | 26.0 ± 1.2 (5.5%) | 27.5 ± 1.3 (11.5%) *** | 27.0 ± 1.3 (9.9%) *** |

The percentage changes with respect to T0 are shown in brackets (i.e. [Tx-TO] / T0).

Legend
*** Tukey-Kramer's p <0.001 vs T0
### p <0.001 Bilateral Student's t test for independent variables (performed on the variation of the data with respect to T0) Active vs Placebo

Digital macrophotography acquired throughout the study provided an objective proof of clinical improvement of the skin affected by AD. A non-invasive technique such as the skin tape stripping was used to measure cytokines expression in skin areas close to those showing AD lesions. With respect to the placebo, TNF alpha, a marker of global of the inflammatory status showed a progressive and statistically significant decrease at T28d and T56d. (Table 18). TARC and TSLP levels, that are specific inflammatory marker of AD lesion, were considerably lower at T28d and T56d (Table 19-20).

**TABLE 18**

| | T0 | T28 | T56 |
|---|---|---|---|
| Active | 108.2 ± 9.3 | 82.5± 8.3 (-23.6%)^{#} | 72.7 ± 6.1 (-32%)^{###} |
| Placebo | 111.1 ± 4.0 | 105.9± 4.1 (-3.0%) | 105.3 ± 3.6 (-3.5) |

The percentage changes with respect to T0 (i.e. [Tx-TO] / T0) are shown in brackets.

Legend:
# p <0.05 - ### p <0.001 Bilateral Student's t test for independent variables (performed on the variation of the data with respect to T0) Active vs Placebo

**TABLE 19**

| | T0 | T28 | T56 |
|---|---|---|---|
| Active | 23.8 ± 0.2 | 22.5 ± 0.2 (-5.6%)^{##} | 22.3 ± 0.1 (-6.2%)^{###} |
| Placebo | 23.1 ± 0.1 | 22.5 ± 0.2 (-2.5%) | 22.7± 0.1 (-1.6%) |

The percentage changes with respect to T0 (i.e. [Tx-TO] / T0) are shown in brackets.

Legend:
## p <0.01 - ### p <0.001 Bilateral Student's t test for independent variables (performed on the variation of the data with respect to T0) Active vs Placebo

**TABLE 20**

| | T0 | T28 | T56 |
|---|---|---|---|
| Active | 28.9 ± 0.5 | 25.6 ± 0.4 (-11.1%)^{###} | 25.9 ± 0.4 (-10.1%)^{###} |
| Placebo | 27.9 ± 0.5 | 27.0 ± 0.5 (-3.4%) | 27.4 ± 0.5 (-1.8%) |

The percentage changes with respect to T0 (i.e. [Tx-TO] / T0) are shown in brackets.

Legend:
### p <0.001 Bilateral Student's t test for independent variables (performed on the variation of the data with respect to T0) Active vs Placebo

A constant improvement in dryness, and desquamation as well as in tightness, itching and burning was observed in the group treated with the composition of Example 1, whereas such symptoms were stable in the placebo group.

Moreover, percentage of responders reached almost a 90% of positive response for tight feeling, skin desquamation and itch intensity at the end of the treatment (T56d) and at the end of the study (T84d).

Indeed, the self-assessment questionnaire revealed that 75% of subjects in the group treated with the composition of Example 1 judged their skin from *quite hydrated* to *well hydrated* after 56 days of treatment, and still 60% of them reported the same answers at the end of the study (T84d). In the placebo group the same answers were respectively 35% and 28%.

The persistence of benefit was perceived for itching too: in the group treated with the composition of Example 1, 70% of subjects judged from "good" to "very good" the reduction of the itching sensation while the remaining 30% perceived this *effect* as "sufficient" at T56d; when interviewed after the end of the study, 85% of subjects declared that such *effect* was maintained from "yes enough" to "yes a lot", while the remaining 15% declared "a little" effect maintenance.

In the placebo group, reduction of the itching sensation was perceived as "good" to "very good" by 38% and as "sufficient" by 45% of subjects at the end of the treatment (T56d), and in terms of maintenance of the effect, 28 days after the end of product intake (T84d), it was judged as "yes enough" and as "a little" by 25% and 40% of subjects respectively.

Administration of the probiotic composition of Example 1 to subjects affected by mild to moderate AD resulted in a statistically significant decrease in SCORAD index. Reduction was already observed after the first 4 weeks of probiotic administration, kept on going throughout the product intake, and lasted even 28 days after the end of intake of the product of the invention.

In this context, an amelioration of such feature is essential to evaluate the general skin beauty benefit. The remarkable achievement obtained with the probiotic treatment was evident not only by the analysis of clinical parameter, but also through comparison of digital photography taken at the beginning, at the end of the treatment and 28 days after the end of treatment.

The probiotic composition of Example 1 provided a continuous positive trend of clinical AD discomforts over time, while symptoms remained relatively stable in the placebo group. Alongside with the dermatologist evaluation, self-assessment questionnaire reported an overall amelioration of AD related symptoms, achieving even better results in the 28 days after the end of product intake, especially for itching and skin hydration.

In summary, the administration of the probiotic composition of Example 1 containing *L. plantarum* PBS067, *L. reuteri* PBS072 and *L. rhamnosus* LRH020 resulted in an amelioration of the AD related symptoms in an adult population.

### Example 10 - Acne

A clinical study was conducted to assess the oral efficacy of the probiotic composition of Example 2 compared to the placebo formulation as reported in Table 21 below in the treatment of acne in adults.

### Materials and method

A randomized double-blind clinical trial, controlled with placebo/reference product (probiotic composition of Example 2) was conducted to assess the effectiveness of a nutraceutical treatment, combined with a cosmetic, improving the appearance of the skin in adult patients affected by acne.

**TABLE 21**

| **INGREDIENTS** | **% p/p** |
|---|---|
| Maltodextrin | 97.96 |
| Magnesium stearate | 1 |
| Silicon dioxide | 1 |
| D-Biotin | 0.04 |

80 female and male subjects (40 subjects per group according to a previously predisposed randomization list) showing a different acne severity from 1 to 3 according to IGA (Investigator's Global Assessment) severity scale have been enrolled. No drop-out occurred.

For ethical reasons, all subjects used a cosmetic product for the treatment of acne. The subjects were then divided homogeneously and randomized into two groups (40 subjects for the active group and 40 for the placebo group) to receive one capsule per day of the probiotic composition shown in Example 2 or of placebo (Table 11) for 56 days.

The instrumental analyses were carried out during the first visit (start - T0), at mid-treatment (28 days - T28) and at the end of treatment (56 days - T56). The study also included clinical evaluation by a dermatologist and a self-assessment questionnaire by volunteers. The visits were carried out in rooms with controlled temperature and humidity (T = 22 2 C and RH = 40-60%). Before the visit the subjects were acclimatised for 15 - 20 minutes.

### Evaluation of skin sebum

The sebum measurement is based on the internationally recognized Sebumeter^{®} method (Sebumeter 815, Courage+Khazaka GmbH). The measurement principle is the photometric method, the grease spot photometer. According to this method the sebum on skin is collected on a 64 mm² mat synthetic tape contained in a cassette, then the measuring head of the cassette is inserted into the aperture of the device, where a photocell measures the transparency of tape due to the sebum presence. The light transmission represents the sebum content on the surface of the measuring area. A microprocessor calculates the result, which is shown on the display in µg sebum/cm² of the skin.

### Evaluation of skin moisturization

The measurement of skin moisturisation is based on the Corneometer^{®} method. Corneometer^{®} method is based on the dielectric constant of water. The probe shows changes of capacitance (dielectric constant of the medium) according to the moisture content of the skin. An electric scatter field penetrates the very first layers of the skin (10-20 µm) and determines the dielectricity, directly proportional to skin hydration. The used device is the Corneometer^{®} CM 825 (Courage+Khazaka, electronic GmbH).

### Evaluation of skin pH

The used instrument is the SKIN pH-METER 905^{®}, Courage + Khazaka GmbH. The measure is based on a combined electrode of high quality, in which both the glass electrode sensitive to H+ and the additional reference electrode are placed in the same site. It is connected to handle probe containing the measurement electronics.

Before the measurements, the SKIN pH-meter^{®} 905 (Courage + Khazaka electronic GmbH) is calibrated using two buffer solutions with known pH (pH 4.01 and 1.7) as reference. Measurement range: 0 to 12. Accuracy: ± 0.1 pH

### Evaluation of the number of acne lesions

The investigator visually assesses (and by palpation if necessary) the facial skin counting the acne lesions. The assessment is mainly based on the evaluation of the number of papules, pustules, open comedones (blackheads) and closed comedones (whiteheads) on the face of the subjects.

### Evaluation of skin complexion evenness

Clinical evaluation of skin complexion evenness is performed by the Dermatologist according to the clinical scores reported in Table 22 below.

**TABLE 22**

| **Clinical classification of skin complexion at T0 (basal evaluation)** | **Score** | **Product effect classification** | **Score** |
|---|---|---|---|
| Skin complexion is uneven, imperfections are all over the face | **1** | No variation | **1** |
| Skin complexion is slightly uneven, imperfections are in some parts of the face | **2** | Slight variation | **2** |
| Skin complexion is quite uniform | **3** | Moderate variation | **3** |
| Skin complexion is uniform | **4** | Evident variation | **4** |

### Evaluation of the improvement of the skin inflammatory status of the area interested by acne lesions

The effect of the product on the improvement of the skin inflammatory status of the area interested by acne lesions is evaluated on the digital pictures acquired with cross-polarized filters. The images are evaluated by the Dermatologist according to the score reported in table 23 below by comparing the images acquired at T0 with those acquired at each following time.

**TABLE 23**

| **Product effect classification** | **Score** |
|---|---|
| No variation | **1** |
| Slight variation | **2** |
| Moderate variation | **3** |
| Evident variation | **4** |

### Digital macrophotography

Digital pictures of the face were acquired at each experimental time using a reflex digital camera (NIKON D300 digital camera, Nikon Corporation Tokyo, Japan) equipped with macro-objective (AF-S Micro NIKKOR 60mm f/2.8G ED, Nikon Corporation Tokyo, Japan), a flash system (Kit R1C1, Nikon Corporation Tokyo, Japan) and cross-/parallel-polarized filters.

### Self-assessment questionnaire

At the end of the study subjects were asked to express their personal opinion on the tested treatment by answering to a questionnaire about products acceptability and effects.

### Results

Both treatment with the probiotic composition reported in Example 2 and that with placebo were well tolerated by the entire study population; no adverse events were recorded during the observation period; finally, no enrolled subject has left the clinical study.

During the entire treatment period, the group that took the probiotic composition reported in Example 2 found a greater reduction in cutaneous sebum both at mid-treatment (T28) and at end of treatment (T56) compared to the basal level, as shown in Table 24.

**TABLE 24**

| | T0 | T28 | T56 |
|---|---|---|---|
| Active | 191.1 ± 18.61 | 168.4±6.97 (-11.8%)* | 146.6 ± 7.18 (-23.0%)* |
| Placebo | 186.1 ± 11.52 | 168.0±9.52 (-9.3%)* | 151.6 ± 8.69 (-18%)* |

The percentage changes with respect to T0 (i.e. [Tₓ-T₀] / T₀) are shown in brackets.

Legend:
* Tukey-Kramer's p <0.05 vs T0

In the same way, also the evaluation of the skin pH, showed a significant reduction in both mid-treatment and prolonged until the end of the product intake. In addition, a significant variation was observed between the group that assumed the probiotic composition reported in Example 2 and the placebo group (-1.78% T0-T28 probiotic composition vs 0% T0-T28 placebo mixture; -3.57% T0-T56 probiotic composition vs 0% T0-T56 placebo mixture; p<0,05 vs placebo) highlighting the contribution of probiotic composition in normalizing skin pH (Table 25).

**TABLE 25**

| | T0 | T28 | T56 |
|---|---|---|---|
| Active | 5.6 ± 0.08 | 5.5 ±0.09 (-1.78%)*^{#} | 5.4 ± 0.10 (-3.57%)*^{#} |
| Placebo | 5.6± 0.11 | 5.6 ± 0.11 (0%) | 5.6 ± 0.12 (0%) |

The percentage changes with respect to T0 are shown in brackets (i.e. [Tx-TO] / T0).

Legend:
* Tukey-Kramer's p <0.05 vs T0
# p <0.05 Bilateral Student's t test for independent variables (performed on the variation of the data with respect to T0) Active vs Placebo

During the 56 days of treatment, the group that took the probiotic composition reported in Example 2 showed a progressive increase in skin hydration as shown in Table 26.

**TABLE 26**

| | T0 | T28 | T56 |
|---|---|---|---|
| Active | 47.1 ± 1.90 | 48.2 ± 2.16 (2.3%) | 49.2 ±2.04 (4.45%)* |
| Placebo | 44.4 ± 1.84 | 45.0 ± 2.04 (1.3%) | 45.1 ± 1.82 (1.5%) |

The percentage changes with respect to T0 are shown in brackets (i.e. [Tₓ-T₀]/T₀).

Legend:
* Tukey-Kramer's p <0.05 vs T0

Evaluation of the number of acneic lesions (papules and pustules) during the treatment period showed a significant reduction already from T28 until the end of T56 treatment (Table 27) in the group that took the probiotic composition reported in Example 2.

**TABLE 27**

| | T0 | T28 | T56 |
|---|---|---|---|
| Active | 11.4 ± 0.83 | 8.7 ± 0.71 (- 2.7)* | 7.4 ± 0.77 (- 4.4)* |
| Placebo | 10.3 ± 0.98 | 9.0 ± 1.27 (- 1.4)* | 6.7 ± 0.99 (- 2.7)* |

The variations with respect to T0 are shown in brackets.

Legend:
* Tukey-Kramer's p <0.05 vs T0

In combination with the number of acneic lesions, the reduction of the inflammatory state surrounding the acneic lesion was also observed expressed as a percentage of subjects who observed an improvement during treatment. The percentage of subjects belonging to the group that took the probiotic composition that observed an improvement at the end of treatment (T56) was 65% against 45% for the placebo group (Table 28).

**TABLE 28**

| | T28 | T56 |
|---|---|---|
| Active | 47.5% | 65.0% |
| Placebo | 37.5% | 45.0% |

In addition, a reduction in the number of both open and closed comedones was observed between the probiotic composition of Example 2 and the placebo group with a visible long-term improvement (T56) as shown in Table 29.

**TABLE 29**

| | T0 | T28 | T56 |
|---|---|---|---|
| Active | 16.6 ± 1.56 | 14.8 ± 1.50 (- 1.8)* | 13 ±1.21 (-3.6)* |
| Placebo | 15.9 ± 1.72 | 14.1 ± 1.37 (- 1.8)* | 13.0 ± 1.86 (- 2.9)* |

The variations with respect to T0 are shown in brackets.

Legend:
* Tukey-Kramer's p <0.05 vs T0

A further parameter observed during the study period was the evaluation of skin complexion, expressed as a percentage of subjects who observed an improvement during treatment with the probiotic composition reported in Example 2 (Table 30). Also in this case the percentage of subjects, belonging to the active group, which had positive results was 67.5% against 45% of the placebo group.

**TABLE 30**

| | T28 | T56 |
|---|---|---|
| Active | 37.5% | 67.5% |
| Placebo | 32.5% | 45.0% |

Finally, the self-assessment questionnaire highlighted a general improvement in the symptoms associated with acne and confirmed a good tolerability of the product of the invention.

In conclusion, the intake of the probiotic composition reported in Example 2 containing *L. plantarum* PBS067, *L. reuteri* PBS072 and *L. rhamnosus* LRH020 showed an improvement in the symptoms associated with acne in an adult population.

## Claims

1. A probiotic composition comprising a combination of the following strains:
- *Lactobacillus plantarum* PBS067,
- *Lactobacillus rhamnosus* LRH020, and
- *Lactobacillus reuteri* PBS072.

2. The probiotic composition according to claim 1, wherein *Lactobacillus plantarum* PBS067 is present in a variable percentage on the total weight of the probiotic combination from 10% to 30%, preferably from 15% to 25%, more preferably it is equal to 20%, or in each single unit dose in amounts ranging from 0.5 to 2.5 billion CFU, preferably in amounts ranging from 1 to 2 billion CFU, more preferably it is present in an amount equal to 1 billion CFU.

3. The probiotic composition according to claim 1, wherein *Lactobacillus rhamnosus* LRH020 is present in a variable percentage on the total weight of the probiotic combination from 25% to 45%, preferably from 30% to 40%, more preferably it is equal to 35% or in each single unit dose in amounts ranging from 0.5 to 2.5 billion CFU, preferably in amounts ranging from 1 to 2 billion CFU, more preferably it is present in an amount equal to 1 billion CFU.

4. The probiotic composition according to claim 1, wherein *Lactobacillus reuteri* PBS072 is present in a variable percentage on the total weight of the probiotic combination from 35% to 55%, preferably from 40% to 50%, more preferably it is equal to 45% or in each single unit dose in amounts ranging from 0.5 to 2.5 billion CFU, preferably in quantities varying from 1 to 2 billion CFU, more preferably it is present in quantities equal to 1 billion CFU.

5. The probiotic composition according to claims 1-4, wherein *Lactobacillus plantarum PBS067:Lactobacillus rhamnosus LRH020:Lactobacillus reuteri* PBS072 are present in a ratio of 1:2.5: 1.75 in percentage on the probiotic combination or equal to 1: 1: 1 if expressed in CFU.

6. The probiotic composition according to claims 1-5, wherein a strain of one of the species *Lactobacillus plantarum* PBS067, *Lactobacillus rhamnosus* LRH020 or *Lactobacillus reuteri* PBS072 is replaced with a strain of the species *Lactobacillus acidophilus*, preferably with the *Lactobacillus acidophilus* strain PBS066.

7. The probiotic composition according to claims 1-6, in oral or topical form.

8. A probiotic composition according to claims 1-7, for use as a medicament.

9. A probiotic composition according to claims 1-7, for use in the prevention and/or treatment of dermatitis and acne.

10. The probiotic composition for use according to claim 9, wherein *Lactobacillus plantarum PBS067:Lactobacillus rhamnosus* LRH020:*Lactobacillus reuteri* PBS072 are present in a ratio of 1:2.5:1.75 in percentage on the probiotic combination or equal to 1:1:1 if expressed in CFU.

11. The probiotic composition for use according to claims 9-10, in oral or topical form.

12. The probiotic composition for use according to claims 9-11, wherein the dermatitis is selected from the group comprising seborrheic dermatitis; rosacea; alopecia; cutaneous vasculitis; eczema; urticaria; angioedema; erythema; vitiligo; preferably from the group comprising: rosacea; cutaneous vasculitis; eczema; urticaria; angioedema; erythema.

13. The probiotic composition for use according to claims 9-11, wherein the dermatitis is selected from the group comprising psoriasis; subacute and chronic, systemic lupus erythematosus; scleroderma; alopecia; dermatomyositis; vulgar or bullous pemphigus; herpetiform dermatitis; linear IgA dermatitis; autoimmune urticaria; vitiligo; preferably from the group comprising: psoriasis; herpetiform dermatitis; autoimmune urticaria.

14. The probiotic composition for use according to claims 9-11, wherein dermatitis is selected from the group comprising atopic dermatitis, contact dermatitis or contact photoallergies; eczema; allergic urticaria; angioedema; erythema; preferably is atopic dermatitis.

## Patentansprüche

1. Probiotische Zusammensetzung, umfassend eine Kombination der folgenden Stämme:
- *Lactobacillus plantarum* PBS067,
- *Lactobacillus rhamnosus* LRH020 und
- *Lactobacillus reuteri* PBS072.

2. Probiotische Zusammensetzung gemäß Anspruch 1, wobei *Lactobacillus plantarum* PBS067 in einem variablen Prozentsatz des Gesamtgewichts der probiotischen Kombination von 10% bis 30%, vorzugsweise von 15% bis 25% vorhanden ist, stärker bevorzugt 20% entspricht, oder in jeder Einzeldosis in Mengen von 0,5 bis 2,5 Milliarden KBE, vorzugsweise in Mengen von 1 bis 2 Milliarden KBE, und noch bevorzugter in einer Menge von 1 Milliarde KBE vorhanden ist.

3. Probiotische Zusammensetzung gemäß Anspruch 1, wobei *Lactobacillus rhamnosus* LRH020 in einem variablen Prozentsatz des Gesamtgewichts der probiotischen Kombination von 25% bis 45%, vorzugsweise von 30% bis 40% vorhanden ist, stärker bevorzugt 35% entspricht, oder in jeder Einzeldosis in Mengen von 0,5 bis 2,5 Milliarden KBE, vorzugsweise in Mengen von 1 bis 2 Milliarden KBE, noch bevorzugter in einer Menge von 1 Milliarde KBE vorhanden ist.

4. Probiotische Zusammensetzung gemäß Anspruch 1, wobei *Lactobacillus reuteri* PBS072 in einem variablen Prozentsatz des Gesamtgewichts der probiotischen Kombination von 35% bis 55%, vorzugsweise von 40% bis 50% vorhanden ist, stärker bevorzugt 45% entspricht, oder in jeder Einzeldosis in Mengen von 0,5 bis 2,5 Milliarden KBE, vorzugsweise in Mengen von 1 bis 2 Milliarden KBE, bevorzugter in einer Menge von 1 Milliarde KBE vorhanden ist.

5. Probiotische Zusammensetzung gemäß den Ansprüchen 1-4, wobei *Lactobacillus plantarum* PBS067:*Lactobacillus rhamnosus LRH020:Lactobacillus reuteri* PBS072 in einem Verhältnis von 1:2,5:1,75 in Prozent der probiotischen Kombination oder gleich 1:1:1, wenn in KBE ausgedrückt, vorhanden sind.

6. Probiotische Zusammensetzung gemäß den Ansprüchen 1-5, wobei ein Stamm einer der Arten *Lactobacillus plantarum* PBS067, *Lactobacillus rhamnosus* LRH020 oder *Lactobacillus reuteri* PBS072 durch einen Stamm der Art *Lactobacillus acidophilus*, vorzugsweise durch den *Lactobacillus acidophilus*-Stamm PBS066, ersetzt ist.

7. Probiotische Zusammensetzung gemäß den Ansprüchen 1-6 in oraler oder topischer Form.

8. Probiotische Zusammensetzung gemäß den Ansprüchen 1-7 zur Verwendung als Medikament.

9. Probiotische Zusammensetzung gemäß den Ansprüchen 1-7 zur Verwendung bei der Vorbeugung und/oder Behandlung von Dermatitis und Akne.

10. Probiotische Zusammensetzung zur Verwendung gemäß Anspruch 9, wobei *Lactobacillus plantarum* PBS067, *Lactobacillus rhamnosus* LRH020 und *Lactobacillus reuteri* PBS072 in einem Verhältnis von 1:2,5:1,75 in Prozent der probiotischen Kombination oder gleich 1:1:1, wenn in KBE ausgedrückt, vorhanden sind.

11. Probiotische Zusammensetzung zur Verwendung gemäß den Ansprüchen 9-10 in oraler oder topischer Form.

12. Probiotische Zusammensetzung zur Verwendung gemäß den Ansprüchen 9-11, wobei die Dermatitis ausgewählt ist aus der Gruppe, umfassend seborrhoische Dermatitis; Rosacea; Alopezie; kutane Vaskulitis; Ekzem; Urtikaria; Angioödem; Erythem; Vitiligo; vorzugsweise aus der Gruppe, umfassend: Rosacea; kutane Vaskulitis; Ekzem; Urtikaria; Angioödem; Erythem.

13. Probiotische Zusammensetzung zur Verwendung gemäß den Ansprüchen 9-11, wobei die Dermatitis ausgewählt ist aus der Gruppe, umfassend Psoriasis; subakuter und chronischer systemischer Lupus erythematodes; Sklerodermie; Alopezie; Dermatomy, vulgärer oder bullöser Pemphigus, herpetiforme Dermatitis, lineare IgA-Dermatitis, Autoimmunurtikaria, Vitiligo, vorzugsweise aus der Gruppe umfassend: Psoriasis, herpetiforme Dermatitis, Autoimmunurtikaria.

14. Probiotische Zusammensetzung zur Verwendung gemäß den Ansprüchen 9-11, wobei die Dermatitis ausgewählt ist aus der Gruppe, umfassend atopische Dermatitis, Kontaktdermatitis oder Kontaktphotoallergien, Ekzem, allergische Urtikaria, Angioödem, Erythem, und vorzugsweise atopische Dermatitis ist.

## Revendications

1. Composition probiotique comprenant une combinaison des souches suivantes :
- *Lactobacillus plantarum* PBS067,
- *Lactobacillus rhamnosus* LRH020, et
- *Lactobacillus reuteri* PBS072.

2. Composition probiotique selon la revendication 1, dans laquelle *Lactobacillus plantarum* PBS067 est présent en un pourcentage variable relativement au poids total de la combinaison probiotique de 10 % à 30 %, de préférence de 15 % à 25 %, plus préférablement il est égal à 20 %, ou dans chaque dose unitaire unique en quantités allant de 0,5 à 2,5 milliards d'UFC, de préférence en quantités allant de 1 à 2 milliards d'UFC, plus préférablement il est présent en une quantité égale à 1 milliard d'UFC.

3. Composition probiotique selon la revendication 1, dans laquelle *Lactobacillus rhamnosus* LRH020 est présent en un pourcentage variable relativement au poids total de la combinaison probiotique de 25 % à 45 %, de préférence de 30 % à 40 %, plus préférablement il est égal à 35 % ou dans chaque dose unitaire unique en quantités allant de 0,5 à 2,5 milliards d'UFC, de préférence en quantités allant de 1 à 2 milliards d'UFC, plus préférablement il est présent en une quantité égale à 1 milliard d'UFC.

4. Composition probiotique selon la revendication 1, dans laquelle *Lactobacillus reuteri* PBS072 est présent en un pourcentage variable relativement au poids total de la combinaison probiotique de 35 % à 55 %, de préférence de 40 % à 50 %, plus préférablement il est égal à 45 % ou dans chaque dose unitaire unique en quantités allant de 0,5 à 2,5 milliards d'UFC, de préférence en quantités variant de 1 à 2 milliards d'UFC, plus préférablement il est présent en quantités égales à 1 milliard d'UFC.

5. Composition probiotique selon les revendications 1 à 4, dans laquelle *Lactobacillus plantarum PBS067:Lactobacillus rhamnosus LRH020-Lactobacillus reuteri* PBS072 sont présents selon un rapport de 1:2,5:1,75 en pourcentage relativement à la combinaison probiotique ou égal à 1:1:1 s'il est exprimé en UFC.

6. Composition probiotique selon les revendications 1 à 5, dans laquelle une souche de l'une des espèces *Lactobacillus plantarum* PBS067, *Lactobacillus rhamnosus* LRH020 ou *Lactobacillus reuteri* PBS072 est remplacée par une souche de l'espèce *Lactobacillus acidophilus,* de préférence par la souche *Lactobacillus acidophilus* PBS066.

7. Composition probiotique selon les revendications 1 à 6, sous forme orale ou topique.

8. Composition probiotique selon les revendications 1 à 7, pour une utilisation en tant que médicament.

9. Composition probiotique selon les revendications 1 à 7, pour une utilisation dans la prévention et/ou le traitement de la dermatite et de l'acné.

10. Composition probiotique pour une utilisation selon la revendication 9, dans laquelle *Lactobacillus plantarum* PBS067:*Lactobacillus rhamnosus* LRH020:*Lactobacillus reuteri* PBS072 sont présents selon un rapport de 1:2,5:1,75 en pourcentage relativement à la combinaison probiotique ou égal à 1:1:1 s'il est exprimé en UFC.

11. Composition probiotique pour une utilisation selon les revendications 9 à 10, sous forme orale ou topique.

12. Composition probiotique pour une utilisation selon les revendications 9 à 11, dans laquelle la dermatite est choisie dans le groupe comprenant la dermatite séborrhéique ; la rosacée ; l'alopécie ; la vascularite cutanée ; l'eczéma ; l'urticaire ; l'œdème de Quincke ; l'érythème ; le vitiligo ; de préférence dans le groupe comprenant : la rosacée ; la vascularite cutanée ; l'eczéma ; l'urticaire ; l'œdème de Quincke ; l'érythème.

13. Composition probiotique pour une utilisation selon les revendications 9 à 11, dans laquelle la dermatite est choisie dans le groupe comprenant le psoriasis ; le lupus érythémateux systémique, subaigu et chronique ; la sclérodermie ; l'alopécie ; la dermatomyosite ; le pemphigus vulgaire ou bulleux ; la dermatite herpétiforme ; la dermatite à IgA linéaire ; l'urticaire auto-immun ; le vitiligo ; de préférence dans le groupe comprenant : le psoriasis ; la dermatite herpétiforme ; l'urticaire auto-immun.

14. Composition probiotique pour une utilisation selon les revendications 9 à 11, dans laquelle la dermatite est choisie dans le groupe comprenant la dermatite atopique, la dermatite de contact ou les photoallergies de contact ; l'eczéma ; l'urticaire allergique ; l'œdème de Quincke ; l'érythème ; est de préférence la dermatite atopique.
